# EUROPEAN PATENT SPECIFICATION

(11) **EP 3 205 312 B1**
(45) Date of publication and mention of the grant of the patent: **12.12.2018**
(21) Application number: 17155225.0
(22) Date of filing: 08.02.2017
(51) Int. Cl.: A61F 2/848, A61F 2/90

(54) **MEDICAL DEVICE WITH TUBULAR BODY HAVING MESH PORTION AND REINFORCEMENT PORTION**
MEDIZINISCHE VORRICHTUNG MIT EINEM NETZABSCHNITT UND VERSTÄRKUNGSABSCHNITT
DISPOSITIF MÉDICAL COMPORTANT UN CORPS TUBULAIRE AYANT UNE PARTIE DE MAILLE ET UNE PARTIE DE RENFORCEMENT

(30) Priority: 11.02.2016 US 201662294035 P
(43) Date of publication of application: 16.08.2017
(73) Proprietor: Cook Medical Technologies LLC, Bloomington, IN 47404 (US)
(72) Inventor: FUREY, Aidan Peter, 2500 Valby (DK); MILNER, Keith R., West Lafayette, IN 47906 (US)
(74) Representative: Simpson, Tobias Rutger

(56) References cited:
- WO-A1-2014/197957
- WO-A2-2013/132478

## Description

### TECHNICAL BACKGROUND

The field of the present disclosure relates to medical devices for deployment in an intraluminal passage and, in particular to flow diverters for treating large neck and fusiform aneurysms.

### BACKGROUND

The statements in this section merely provide background information related to the present disclosure and may not constitute prior art.

Flow diverters are used to treat large neck and fusiform aneurysms. Flow diverters typically are deployed within an intraluminal passage and include a dense mesh which blocks flow of blood into the aneurysm. However, typically, to prevent blood flow, small wires must be used in forming the flow diverter to achieve an outer surface mesh with sufficiently narrow openings. Flow diverters made with such small wires may have a low radially expansive force. This may be undesirable. A prior art device is shown in WO 2013/132478.

### SUMMARY

Insofar as the terms "invention" and/or "embodiment" are used in the following, and/or features are presented as being optional, this should be interpreted in such a way that the only protection sought is that of the invention as claimed. Further areas of applicability will become apparent from the description provided herein. It should be understood that the description and specific examples are intended for the purpose of illustration only and are not intended to limit the scope of the present disclosure.

It is desirable to provide flow diverter which is capable of preventing the flow of blood into the aneurysm while preventing migration and buckling.

In one form of the present disclosure, a medical device is provided including a tubular body which is radially expandable. The medical device is configured to be positioned within an intraluminal passage. The tubular body includes multiple first wires braided together and extends from a first end to a second end. The tubular body has a reinforcement region and a mesh region. The mesh region is disposed between the first and second ends and includes multiple second wires braided with the first wires. Within the reinforcement region, at least one of the first wires is folded onto one of the first and second wires. The mesh region may be capable of preventing the flow of blood into an aneurysm and the reinforcement region may be capable of preventing migration and buckling.

In another form of the present disclosure, a medical device is provided including a tubular body and a mesh region. The medical device is configured to be positioned within an intraluminal passage. The tubular body is radially expandable and includes first wires which are braided together. The tubular body extends from a first end to a second end. The mesh region is disposed on the tubular body between the first and second ends. The mesh region includes multiple second wires which are braided with the first wires. The thickness of the first wires is greater than the thickness of the second wires. At least of the first wires of the tubular body is reinforced by at least one of the first wires being folded onto one of the first and second wires.

In yet another form of the present disclosure, a method of manufacturing a medical device is provided including forming a tubular body, forming a mesh region, and reinforcing a portion of the tubular body. The medical device is configured to be positioned within an intraluminal passage. The tubular body is radially expandable, extends between a first end and a second end, and is formed by braiding together multiple first wires. The mesh region is formed between the first and second ends of the tubular body. The mesh region is formed by braiding multiple second wires with the first wires. A thickness of the first wires is greater than a thickness of the second wires. A portion of the tubular body is reinforced by folding at least one of the first wires onto another of the first and second wires.

### BRIEF DESCRIPTION OF THE DRAWINGS

The disclosure may be more fully understood by reading the following description in conjunction with the drawings, in which:
FIG. 1 is a side plan view of a first example of a medical device including a mesh region, reinforcement regions, and intermediate regions;
FIG. 2 is partial side plan view of a reinforcement region of the first example of the medical device;
FIG. 3 is a side plan view of a second example of a medical device including a mesh region and reinforcement regions;
FIG. 4 is a partial side plan view of a reinforcement region of the second example of the medical device; and
FIG. 5 is a flow chart depicting a method of manufacturing a medical device.

The drawings described herein are for the purpose of illustration only and are not intended to limit the scope of the present disclosure in any way.

### DETAILED DESCRIPTION

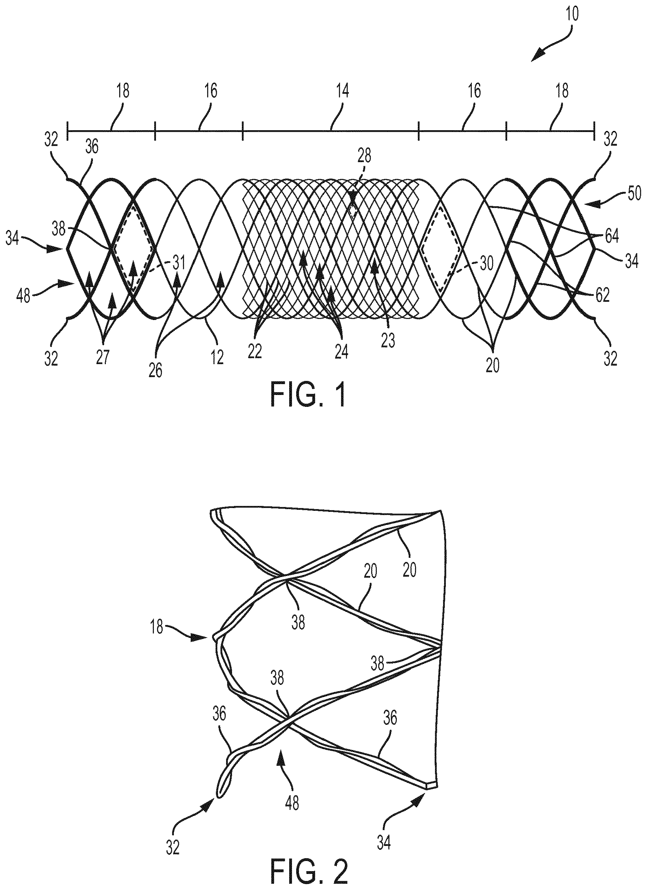

Referring now to the drawings, and particularly to FIG. 1, a medical device 10 is shown which is preferably radially expandable. In this embodiment, the medical device includes a tubular body 12 which is cylindrical and extends between first and second ends 48, 50. As shown, the tubular body 12 has a constant diameter between the first and second ends 48, 50. Alternatively, the first and second ends 48, 50 may flare outwardly. In this embodiment, the tubular body 12 includes an intermediate region 16 and a reinforcement region 18. The intermediate region 16 comprises a mesh region 14. The tubular body 12 includes a plurality of first wires 20 which extend along the entire length of the tubular body 12 and are braided together. In this embodiment, the first wires 20 are braided together such that each of the first wires 20 extends from the first end 48 to the second end 50 of the tubular body 12. As the first wires 20 extend along the length of the tubular 12, a clockwise portion 62 of the first wires 20 curve in a clockwise direction about the circumference of the tubular body 12. A counter-clockwise portion 64 of the first wires 20 curve in a counter-clockwise direction about the circumference of the tubular body 12. Each of the clockwise portion 62 of first wires 20 intersect with at least one of the counter-clockwise portion 64 of first wires 20 while extending along the length of the tubular body. Where these intersections occur, each of the clockwise portion 62 of the first wires 20 passes over or under the at least one counter-clockwise 64 first wire 20 in an alternating pattern. For example, a clockwise 62 first wire 20 extend from the first end 48 of the tubular body, and may pass over a first counter-clockwise 64 first wire 20 at a first intersection, pass under a second counter-clockwise 64 first wire 20 at a second intersection, and pass over a third counter-clockwise 64 first wire 20 at a third intersection, continuing in this pattern until reaching the second end 50 of the tubular body 12.

The tubular body 12 may be moved between a compressed configuration and an expanded configuration. The tubular body 12 may be heat set in the expanded position to create a radially expansive force in the first wires 20 when the tubular body 12 is in the compressed configuration.

In this embodiment, the mesh region 14 includes the plurality of second wires 22 braided with the plurality of first wires 20 and is disposed within the intermediate region 16. However, the mesh region 14 may be disposed along any portion of the tubular body 12 to minimize the flow of blood through openings 24 in a side wall 23 of the tubular body 12. Thus, the mesh region 14 may be located at any position along the length of the tubular body 12 between the first and second ends 48, 50. Moreover, the mesh region 14 may extend along the entire length of the tubular body 12.

The second wires 22 of the mesh region 14 are braided in a dense arrangement with the first wires 20. The density of the braided second wires 22 in the mesh region is sufficient to minimize the flow of blood passing from an interior of the tubular body 12 and through the outer surface of the tubular body. In one possible use of the medical device 10, the mesh region 14 is positioned to partially or entirely cover an aneurysm within an intraluminal passage. In such an embodiment, the tubular body 12 is pressed against the walls of the intraluminal passage, allowing most or all of the blood passing through the intraluminal passage to pass through the interior of the tubular body 12. The density of the braided first wires 20 and second wires 22 in the mesh region 14 minimizes blood flow into the aneurysm, thereby preventing further strain on the aneurysm and/or allowing the aneurysm to heal.

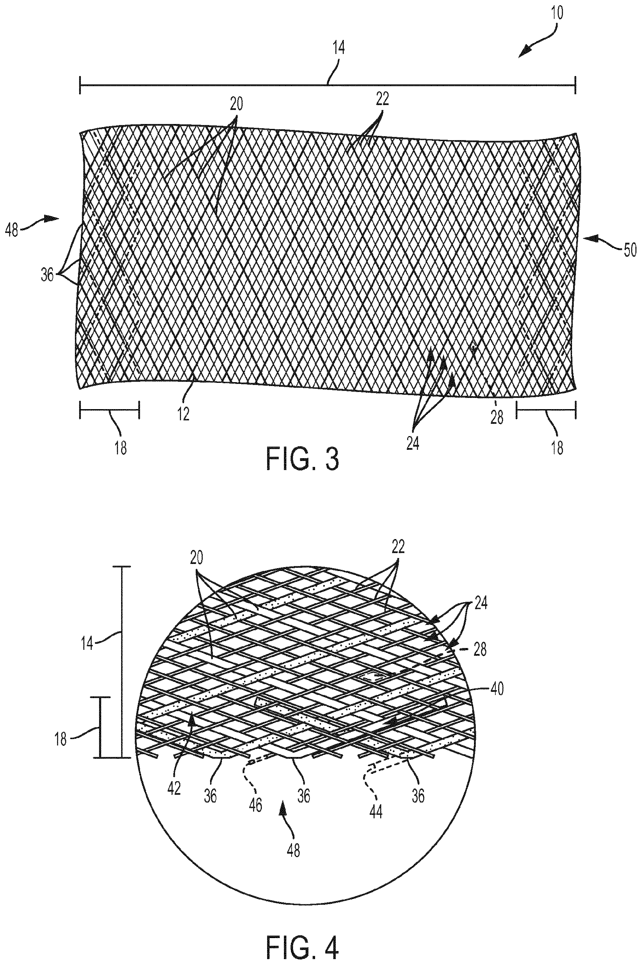

In this embodiment, the thickness (44 in FIG. 4) of the first wires 20 is between about (0.033mm and 0.043mm) 0.0013 inches and 0.0017 inches, and preferably between (0-033mm and 0.043mm) 0.0013 inches and 0.0017 inches. Comparatively, the second wires 22 have smaller thickness (46 in FIG. 4) between about (0.015mm and 0.025mm) 0.0006 inches and 0.001 inches, and preferably between (0.015mm and 0.025mm) 0.0006 inches and 0.001 inches. As a result, the first wires 20 contribute a greater radially expansive force to the tubular body 12, but may be too large to minimize the flow of blood through openings 24 in the side wall 23 of the mesh region 14. Due to the smaller thickness, the smaller second wires 22 may provide less radially expansive force than the first wires 20. However, the second wires 22 are braided together with the first wires 20 in the mesh region to minimize the flow of blood through the openings 24 in the side wall 23 at the mesh region 14.

The first wires 20 may be made from any material which would provide a radially expansive force to the tubular body 12, such as stainless steel or another metal alloy, or any other suitable material. An alloy which is capable of being heat set into the expanded configuration, such as nitinol, may also be used. The second wires 22 may be made from any material which may be braided to form the mesh region 14 with openings 24 in the side wall of the tubular body 12. The second wires 22 may also contribute some smaller radially expansive force relative to the first wires 20 and may be made of a metal or metal alloy such as stainless steel or nitinol, or any other suitable material.

In this embodiment, there are more second wires 22 than first wires 20 in the mesh region 14. However, it is to be understood that there may be more or less second wires 22 than first wires 20 within the mesh region 14 without departing from the spirit of the present invention. The ratio between the number of second wires 22 to the number of first wires 20 may be between three and fifteen. Therefore, the total number of first wires 20 in the tubular body 12 may be between about 6% and 25% of the total number of first wires 20 and second wires 22 in the tubular body 12, and preferably between 6% and 25% of the total number of first wires 20 and second wires 22 in the tubular body 12. The sum of the first wires 20 and second wires 22 may be between sixty-four and one-hundred-and-twenty-eight. In other embodiments, the total number of wires may be divisible by eight for ease of construction. For example, an embodiment may have sixty-four total wires, with sixteen first wires 20 and forty-eight second wires 22. Another embodiment may have one-hundred-and-twenty-eight wires, with eight first wires 20 and one-hundred-and-twenty second wires 22. A higher the ratio of second wires 22 to first wires 20 may result in a better sealed mesh region 14, while a lower ratio of second wires 22 to first wires 20 may result in a tubular body 12 having greater radial expansive force.

In this embodiment, the reinforcement region 18 is any region on the tubular body 12, wherein the reinforcement region 18 comprises a greater radially expansive force relative to any the intermediate region 16. In addition, the reinforcement region 18 is defined by having a folded portion 36 as further described in greater detail below. In this embodiment, the reinforcement region 18 is located both of the first end 48 and second end 50. The reinforcement region 18 may overlap with the mesh region 14. Each reinforcement region 18 may extend along the length of the tubular body 12 between about 0.04 inches and 0.2 inches, and preferably between 0.06 inches and 0.12 inches, or between about 5% and 10%, preferably between 5% and 10% of the total length of the tubular body 12.

In other embodiments, the reinforcement region 18 may be located only at one of the first and second ends 48, 50. Blood flow downstream through the intraluminal passage may cause migration of the medical device 10 only in a downstream direction. Therefore, a single reinforcement region 18 disposed on only one of the first and second ends 48, 50 of the tubular body 12 which is upstream of the blood flow may prevent migration. In other embodiments, a single reinforcement region 18 disposed on only one of the first and second ends 48, 50 which is downstream of the blood flow may be preferable. However, reinforcement regions 18 on both the first and second ends 48, 50 may provide more anchoring force in an intraluminal passage.

In this embodiment, within the reinforcement region 18, at least one of the first wires 20 is folded onto one of the first and second wires 20, 22. In some embodiments, all of the first wires 20 may be folded onto first and second wires 20, 22, defining a folded portion 36 of the first wire 20. As shown, the folded portion 36 overlaps with the other portions of the first wires 20 within the reinforcement region 18, increasing the radially expansive force in the reinforcement region 18. Therefore, the folding of the first wires 20 may increase the radially expansive force of the reinforcement region 18. For this reason, the reinforcement region 18 may assist in anchoring the medical device 10 against the walls of an intraluminal passage (not shown). The first wires 20 may be folded at either of the first and second ends 48, 50 of the tubular body 12. The first wires 20 may also be folded at a position between the first and second ends 48, 50 of the tubular body 12.

In one embodiment shown in FIGS. 1 and 2, the first wires 20 have the folded portion 36 of the at least one of the first wires 20 fold onto and then twist about the same first wire 20, forming a self-folded end 32. The self-folded end 32 has a twist which extends inwardly along the length of the tubular body 12. The twist of the self-folded end 32 extends back along the first wire 20 for a distance of between about (1mm and 6.35mm) 0.04 inches and 0.25 inches, preferable between (1.59mm and 4.76mm) 0.0625 inches and 0.1875 inches. The twist of the self-folded end 32 may provide additional radially expansive force to the reinforcement region 18 by twisting back over at least one braided intersection 38 of braided first wires 20. This may be particularly effective where multiple self-folded ends 32 are folded through the braided intersection 38 forming a complex and strong braided intersection 38. The folded portion 36 of the self-folded ends 32 may extend though between one and five braided intersections 38.

In this embodiment shown in FIGS 1 and 2, at least one of the first wires 20 is folded onto another adjacent first wire 20 and then twisted about the adjacent first wire 20, forming an adjacent-folded end 34. The adjacent-folded end 34 may be utilized in embodiments where other folds of the first wire 20 are not structurally practical. Similar to the self-folded end 32, the adjacent-folded end 34 has a twist which extends back along the first wire 20 for a distance of between about (1mm and 6.35mm) 0.04 inches and 0.25 inches, preferable between (1.59mm and 4.76mm) 0.0625 inches and 0.1875 inches. The twist of the adjacent-folded end 34 may be folded and twisted through between one and five braided intersections 38.

In the embodiment shown in FIGS 1 and 2, the tubular body 12 also includes an intermediate region 16 spaced apart from the reinforcement region 18. The intermediate region may include all of or a portion of the mesh region 14. In other embodiments, the intermediate region 16 may not be present. In this embodiment, the intermediate region 16 includes first wires 20 along a length of the tubular body 12 where the first wires 20 have not been folded. Where the intermediate region 16 does not overlap with the mesh region 14, the intermediate region 16 may have openings 26 which are substantially larger than the openings 24 in the mesh region 14. Similarly, where the reinforcement region 18 does not overlap with the mesh region 14, the reinforcement region may have openings 27 which are substantially larger than the openings 24 in the mesh region 14. The openings 24 in the mesh region 14 may have a cross-sectional area 28 between about (0.0006 square mm and 0.26 square mm) 0.000001 square inches and 0.0004 square inches, and preferably between (0.0026 square mm and 0.1 square mm) 0.000004 square inches and 0.00016 square inches. Comparatively, where the reinforcement region 18 does not overlap with the mesh region, the openings 27 in the reinforcement region 18 may have a cross-sectional area 31 between about (0.077 square mm to 45.2 square mm) 0.00012 square inches and 0.07 square inches, preferably between (0.3 square mm and 11.6 square mm) 0.00046 square inches and 0.018 square inches. The openings 26 in the intermediate region 16 may have a cross-sectional area 30 which is the same or larger than the openings 24 in the mesh region 18. Similarly the openings 26 in the intermediate region 16 may have a cross-sectional area 30 which is the same or smaller than the openings 24 in the reinforced region 18. The larger openings 26 in the intermediate region 16 may not be able to prevent the flow of blood through the outer surface of the tubular body 12. Therefore, the medical device 10 may be positioned such that the mesh region 14 covers the entire treatment area within the intraluminal passage.

In yet another embodiment as shown in FIGS. 3 and 4, the mesh region 14 extends along the entire length of the tubular body 12, between the first and second ends 48, 50. Furthermore, in this embodiment, the mesh region 14 overlaps with the reinforcement regions 18. Where such overlap between the mesh region 14 and the reinforcement region 18 is present, another method of folding the first wires 20 in the reinforcement region 18 may be utilized. As shown in FIGS. 3 and 4, the first wires 20 are folded onto one of the first and second wires 20, 22 and braided into the mesh region 14. As shown in FIG. 4, the folded portion 36 of the first wires 20 are braided back into the mesh region 14 passing over 40 and under 42 alternating wires. The folded portion 36 of the first wires 20 proceed over 40 and under 42 only the first wires 20. Alternatively, the folded portion 36 may proceed over 40 and under 42, alternating, any of the first and second wires 20, 22. The folded portion 36 of the first wires 20 may proceed over 40 and under 42 between two and ten of the first and second wires 20, 22.

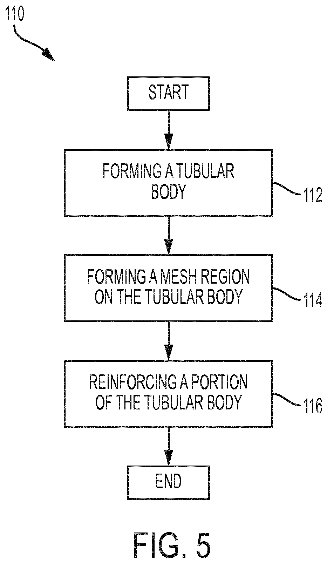

FIG. 5 illustrates a flow chart 110 depicting a method of manufacturing the medical device in accordance with one example of the present invention. As shown in FIG. 5, the method comprises forming the tubular body (112). The tubular body may be formed (112) by braided a multiple first wires together from a first end to a second end.

**The** method further comprises forming a mesh region on the tubular body (114). The mesh region may be formed (114) on the tubular body by braiding multiple second wires with the first wires of the tubular body. The mesh region may be formed (114) anywhere between the first and second ends.

The method further comprises reinforcing a portion of the tubular body (116). Reinforcing the portion of the tubular body (116) may occur before or after forming the mesh region on the tubular body (114). Reinforcing the portion of the tubular body (116) may involve folding at least one of the first wires onto one of the first and second wires, forming a folded portion. The first wire may be folded onto the same first wire and twisted about the same first wire. The first wire may also be folded onto and twisted about an adjacent first wire. The first wire may also be folded onto one of the first and second wires and braided into the mesh region.

The method may also include the step of heat setting the tubular body in the expanded configuration. Heat setting the tubular body ensures that the medical device may be self-expanding and may occur at any point in the method. For example, one or more of the first wires may be heat set after immediately after forming the tubular body (112). Alternately, heat setting may occur after the entire medical device has been assembled, heat setting both the first wires and the second wires while the medical device is in the expanded configuration.

Accordingly, it is now apparent that there are many advantages provided herein. In addition to the advantages that have been described, it is also possible that there are still other advantages that are not currently recognized but which may become apparent at a later time.

While preferred embodiments have been described, it should be understood that the invention is not so limited, and modifications may be made without departing from the invention. The scope of the invention is defined by the appended claims, and all devices that come within the meaning of the claims, either literally or by equivalence, are intended to embrace them.

## Claims

1. A medical device (10) configured to be positioned within an intraluminal passage, the device comprising:
a tubular body (12) being radially expandable, the tubular body comprising a plurality of first wires (20) braided together, the tubular body having a first end extending to a second end, the tubular body having a mesh region (14) disposed between the first and second ends, the mesh region comprising a plurality of second wires (22) braided with the plurality of first wires, (20) **characterised in that** the tubular body has a reinforcement region (18) adjacent one of the first and second ends, the reinforcement region (18) having at least one of the first wires (20) being folded onto one of the first (20) and second (22) wires to prevent migration of the tubular body within the intraluminal passage.

2. The medical device of Claim 1, wherein a thickness of the first wires is greater than a thickness of the second wires.

3. The medical device of Claim 1 or Claim 2, wherein reinforcement regions are located at both the first end and the second end of the tubular body.

4. The medical device of any one of the preceding claims, wherein the tubular body has an intermediate region positioned apart from the reinforcement region.

5. The medical device of any one of the preceding claims, wherein at least one of the first wires is folded onto and twisted about the same first wire.

6. The medical device of any one of the preceding claims, wherein at least one of the first wires is folded onto toward and twisted about an adjacent first wire.

7. The medical device of any one of the preceding claims, wherein at least one of the first wires is folded onto at least one of the first and second wires and braided into the mesh region.

8. The medical device of any one of the preceding claims, wherein the reinforcement region comprises no more than 10% of a total length of the tubular body.

9. The medical device of any one of the preceding claims, wherein a total number of first wires in the tubular body is between 6% and 25% of a total number of first wires and second wires in the tubular body.

10. The medical device of any one of the preceding claims, wherein at least one of the first wires is folded onto and twisted about an adjacent first wire such that a folded portion of the first wire extends beyond at least one intersection of two braided first wires.

11. The medical device of any one of the preceding claims, wherein at least one of the first wires is folded onto at least one of the first and second wires and braided into the mesh region, preferably wherein at least one of the first wires is folded and braided into the mesh region such that a folded portion of the first wire passes over at least one of the first and second wires in the mesh region and under at least another of the first and second wires in the mesh region.

12. A method of manufacturing a medical device configured to be positioned within an intraluminal passage comprising:
forming a tubular body comprising a first end extending to a second end, and being radially expandable by braiding together a plurality of first wires between the first and second ends;
forming a mesh region on the tubular body between the first and second ends by braiding a plurality of second wires with the plurality of first wires wherein a thickness of the first wires is greater than a thickness of the second wires; and
reinforcing a portion of the tubular body by folding at least one of the first wires onto one of the first and second wires.

13. The method of Claim 12, further comprising folding at least one of the first wires onto the same first wire and twisting a folded portion of the first wire about the same first wire, and/or folding at least one of the first wires onto an adjacent first wire and twisting a folded portion of the first wire about the adjacent first wire.

14. The method of any one of Claim 12 to Claim 13, further comprising folding at least one of the first wires onto one of the first and second wires and braiding a folded portion of the first wire into the mesh region.

15. The method of any one of Claim 12 to Claim 14, further comprising heating at least one of the first wires such that the tubular body is heat set in an expanded configuration.

## Patentansprüche

1. Medizinische Vorrichtung (10), die zur Positionierung in einem intraluminalen Durchgang ausgebildet ist, wobei die Vorrichtung Folgendes umfasst:
einen radial expandierbaren röhrenförmigen Körper (12), wobei der röhrenförmige Körper eine Vielzahl von miteinander verflochtenen ersten Drähten (20) umfasst, wobei der röhrenförmige Körper ein sich zu einem zweiten Ende erstreckendes erstes Ende hat, wobei der röhrenförmige Körper einen zwischen dem ersten und dem zweiten Ende angeordneten Netzbereich (14) hat, wobei der Netzbereich eine Vielzahl von mit der Vielzahl von ersten Drähten (20) verflochtenen zweiten Drähten (22) umfasst, **dadurch gekennzeichnet, dass** der röhrenförmige Körper einen Verstärkungsbereich (18) in der Nähe des ersten oder des zweiten Endes hat, wobei bei dem Verstärkungsbereich (18) mindestens einer der ersten Drähte (20) auf einen der ersten (20) und zweiten (22) Drähte gefaltet ist, um eine Migration des röhrenförmigen Körpers in dem intraluminalen Durchgang zu verhindern.

2. Medizinische Vorrichtung nach Anspruch 1, wobei eine Dicke der ersten Drähte größer als eine Dicke der zweiten Drähte ist.

3. Medizinische Vorrichtung nach Anspruch 1 oder Anspruch 2, wobei Verstärkungsbereiche sowohl am ersten Ende als auch am zweiten Ende des röhrenförmigen Körpers angeordnet sind.

4. Medizinische Vorrichtung nach einem der vorhergehenden Ansprüche, wobei der röhrenförmige Körper einen vom Verstärkungsbereich getrennt positionierten Zwischenbereich hat.

5. Medizinische Vorrichtung nach einem der vorhergehenden Ansprüche, wobei mindestens einer der ersten Drähte auf denselben ersten Draht gefaltet und um ihn herum gewunden ist.

6. Medizinische Vorrichtung nach einem der vorhergehenden Ansprüche, wobei mindestens einer der ersten Drähte auf einen benachbarten ersten Draht zu gefaltet und um ihn herum gewunden ist.

7. Medizinische Vorrichtung nach einem der vorhergehenden Ansprüche, wobei mindestens einer der ersten Drähte auf mindestens einen der ersten und zweiten Drähte gefaltet und in den Netzbereich geflochten ist.

8. Medizinische Vorrichtung nach einem der vorhergehenden Ansprüche, wobei der Verstärkungsbereich höchstens 10 % einer Gesamtlänge des röhrenförmigen Körpers umfasst.

9. Medizinische Vorrichtung nach einem der vorhergehenden Ansprüche, wobei eine Gesamtzahl von ersten Drähten im röhrenförmigen Körper zwischen 6 % und 25 % einer Gesamtzahl erster und zweiter Drähte im röhrenförmigen Körper beträgt.

10. Medizinische Vorrichtung nach einem der vorhergehenden Ansprüche, wobei mindestens einer der ersten Drähte auf einen benachbarten ersten Draht gefaltet und um ihn herum gewunden ist, so dass sich ein gefalteter Abschnitt des ersten Drahts über mindestens einen Schnittpunkt zweier geflochtener erster Drähte hinaus erstreckt.

11. Medizinische Vorrichtung nach einem der vorhergehenden Ansprüche, wobei mindestens einer der ersten Drähte auf mindestens einen der ersten und zweiten Drähte gefaltet und in den Netzbereich geflochten ist, vorzugsweise wobei mindestens einer der ersten Drähte gefaltet und in den Netzbereich geflochten ist, so dass ein gefalteter Abschnitt des ersten Drahts über mindestens einen der ersten und zweiten Drähte im Netzbereich und unter mindestens einen anderen der ersten und zweiten Drähte im Netzbereich geht.

12. Verfahren zur Herstellung einer medizinischen Vorrichtung, die zur Positionierung in einem intraluminalen Durchgang ausgebildet ist, umfassend:
Bilden eines röhrenförmigen Körpers, der ein sich zu einem zweiten Ende erstreckendes erstes Ende umfasst und radial expandierbar ist, indem eine Vielzahl von ersten Drähten zwischen dem ersten und dem zweiten Ende miteinander verflochten werden,
Bilden eines Netzbereichs am röhrenförmigen Körper zwischen dem ersten und dem zweiten Ende durch Verflechten einer Vielzahl von zweiten Drähten mit der Vielzahl von ersten Drähten, wobei eine Dicke der ersten Drähte größer als eine Dicke der zweiten Drähte ist, und
Verstärken eines Abschnitts des röhrenförmigen Körpers durch Falten mindestens eines der ersten Drähte auf einen der ersten und zweiten Drähte.

13. Verfahren nach Anspruch 12, ferner umfassend Falten mindestens eines der ersten Drähte auf denselben ersten Draht und Winden eines gefalteten Abschnitts des ersten Drahts um denselben ersten Draht und/oder Falten mindestens eines der ersten Drähte auf einen benachbarten ersten Draht und Winden eines gefalteten Abschnitts des ersten Drahts um den benachbarten ersten Draht.

14. Verfahren nach einem von Anspruch 12 bis Anspruch 13, ferner umfassend Falten mindestens eines der ersten Drähte auf einen der ersten und zweiten Drähte und Flechten eines gefalteten Abschnitts des ersten Drahts in den Netzbereich.

15. Verfahren nach einem von Anspruch 12 bis Anspruch 14, ferner umfassend Erhitzen mindestens eines der ersten Drähte, so dass der röhrenförmige Körper in einer expandierten Konfiguration heißverfestigt wird.

## Revendications

1. Dispositif médical (10) configuré pour être positionné à l'intérieur d'un passage intraluminal, le dispositif comprenant :
un corps tubulaire (12) dilatable radialement, le corps tubulaire comprenant une pluralité de premiers fils (20) tressés les uns avec les autres, le corps tubulaire comportant une première extrémité s'étendant jusqu'à une seconde extrémité, le corps tubulaire comportant une région en résille (14) disposée entre les première et seconde extrémités, la région en résille comprenant une pluralité de seconds fils (22) tressés avec la pluralité de premiers fils (20), **caractérisé en ce que** le corps tubulaire comporte une région de renforcement (18) adjacente à la première ou la seconde extrémité, la région de renforcement (18) comportant au moins un des premiers fils (20) qui est replié sur un des premiers (20) et seconds (22) fils afin d'empêcher une migration du corps tubulaire à l'intérieur du passage intraluminal.

2. Dispositif médical selon la revendication 1, dans lequel une épaisseur des premiers fils est supérieure à une épaisseur des seconds fils.

3. Dispositif médical selon la revendication 1 ou la revendication 2, dans lequel des régions de renforcement sont situées au niveau à la fois de la première extrémité et de la seconde extrémité du corps tubulaire.

4. Dispositif médical selon l'une quelconque des revendications précédentes, dans lequel le corps tubulaire comporte une région intermédiaire positionnée séparément de la région de renforcement.

5. Dispositif médical selon l'une quelconque des revendications précédentes, dans lequel au moins un des premiers fils est replié sur ledit premier fil et entortillé autour de lui.

6. Dispositif médical selon l'une quelconque des revendications précédentes, dans lequel au moins un des premiers fils est replié en direction d'un premier fil adjacent et entortillé autour de celui-ci.

7. Dispositif médical selon l'une quelconque des revendications précédentes, dans lequel au moins un des premiers fils est replié sur au moins un des premiers et seconds fils et tressé dans la région en résille.

8. Dispositif médical selon l'une quelconque des revendications précédentes, dans lequel la région de renforcement constitue au plus 10 % d'une longueur totale du corps tubulaire.

9. Dispositif médical selon l'une quelconque des revendications précédentes, dans lequel un nombre total de premiers fils dans le corps tubulaire est compris entre 6 % et 25 % d'un nombre total de premiers fils et de seconds fils dans le corps tubulaire.

10. Dispositif médical selon l'une quelconque des revendications précédentes, dans lequel au moins un des premiers fils est replié sur un premier fil adjacent et entortillé autour de celui-ci de telle sorte qu'une partie repliée du premier fil s'étende au-delà d'au moins une intersection de deux premiers fils tressés.

11. Dispositif médical selon l'une quelconque des revendications précédentes, dans lequel au moins un des premiers fils est replié sur au moins un des premiers et seconds fils et tressé dans la région en résille, de préférence dans lequel au moins un des premiers fils est replié et tressé dans la région en résille de telle sorte qu'une partie repliée du premier fil passe pardessus au moins un des premiers et seconds fils dans la région en résille et par-dessous au moins un autre des premiers et seconds fils dans la région en résille.

12. Procédé de fabrication d'un dispositif médical configuré pour être positionné à l'intérieur d'un passage intraluminal comprenant :
former un corps tubulaire comprenant une première extrémité s'étendant jusqu'à une seconde extrémité, et qui est dilatable radialement, en tressant les uns avec les autres une pluralité de premiers fils entre les première et seconde extrémités ;
former une région en résille sur le corps tubulaire entre les première et seconde extrémités en tressant une pluralité de seconds fils avec la pluralité de premiers fils, une épaisseur des premiers fils étant supérieure à une épaisseur des seconds fils ; et
renforcer une partie du corps tubulaire en repliant au moins un des premiers fils sur un des premiers et seconds fils.

13. Procédé selon la revendication 12, comprenant en outre le fait de replier au moins un des premiers fils sur ledit premier fil et d'entortiller une partie repliée du premier fil autour dudit premier fil, et/ou de replier au moins un des premiers fils sur un premier fil adjacent et d'entortiller une partie repliée du premier fil autour du premier fil adjacent.

14. Procédé selon l'une ou l'autre de la revendication 12 et la revendication 13, comprenant en outre le fait de replier au moins un des premiers fils sur un des premiers et seconds fils et de tresser une partie repliée du premier fil dans la région en résille.

15. Procédé selon l'une quelconque des revendications 12 à 14, comprenant en outre le fait de chauffer au moins un des premiers fils de telle sorte que le corps tubulaire soit thermofixé dans une configuration dilatée.
